# FASCICULE DE BREVET EUROPEEN

(11) **EP 0 357 755 B1**
(45) Date de publication et mention de la délivrance du brevet: **11.08.1993**
(21) Numéro de dépôt: 89903498.7
(22) Date de dépôt: 15.03.1989
(51) Int. Cl.: A61L 31/00

(54) **PATCH DE CHIRURGIE VISCERALE**
PATCH FÜR EINGEWEIDECHIRURGIE
PATCH FOR VISCERAL SURGERY

(30) Priorité: 15.03.1988 FR 8803321
(43) Date de publication de la demande: 14.03.1990
(73) Titulaire: IMEDEX, F-69007 Lyon (FR); PASTEUR MERIEUX SERUMS ET VACCINS, 69007 Lyon (FR)
(72) Inventeur: TAYOT, Jean-Louis, F-69890 La Tour-de-Salvagny (FR); MARESCAUX, Jacques, F-67310 Scharrachbergheim (FR); DUMAS, Henri, Bât. 11A F-69005 Lyon (FR); TARDY, Michel, F-69005 Lyon (FR)
(74) Mandataire: Lemoine, Michel
(86) Numéro de dépôt international: FR8900108
(87) Numéro de publication internationale: WO8908467

(56) Documents cités:
- EP-A- 0 042 253
- EP-A- 0 213 563
- EP-A- 0 227 955
- DE-A- 1 492 300
- DE-A- 2 348 685
- FR-A- 1 458 786

## Description

La présente invention a trait à l'application d'un biomatériau à base de collagène à la réalisation d'un patch de chirurgie viscérale.

On rencontre fréquemment, en chirurgie viscérale, des accidents post-opératoires résultant d'un défaut de cicatrisation des viscères. Or, il faut savoir que l'absence de cicatrisation des viscères est la principale source de mortalité, de morbidité et de surcoût en chirurgie viscérale et en particulier digestive.

Pourtant, il n'a pas encore été trouvé de solution satisfaisante à ce problème de cicatrisation, problème qui se trouve exacerbé lorsque le contexte est défavorable, par exemple en cas de fistule digestive, de milieu septique ou encore d'agression chimique, notamment agression biliaire, etc.

Alors que diverses spécialités chirurgicales bénéficient largement de biomatériaux sans cesse perfectionnés, la chirurgie viscérale et en particulier digestive a délaissé cette voie.

La présente invention vise à résoudre ce problème, en proposant un patch de chirurgie viscérale réalisé à partir d'un biomatériau, qui permette une bonne cicatrisation des viscères et cela, même dans des conditions défavorables.

Un autre objectif de l'invention est de fournir un patch de chirurgie viscérale, réalisé à partir d'un biomatériau qui se colle facilement.

Un autre objectif encore de l'invention est de fournir un patch de chirurgie viscérale, réalisé à partir d'un biomatériau entièrement biodégradable et qui réalise un excellent effet de confinement, d'hémostase, en remplaçant temporairement la paroi à reconstituer.

Un autre objectif encore de l'invention est de proposer un patch de chirurgie viscérale réalisé à partir d'un biomatériau à base de composés naturellement présents dans les tissus et subissant un minimum de transformations.

Un autre objectif encore de l'invention est de fournir un patch de chirurgie viscérale réalisé à partir d'un biomatériau résistant mécaniquement aussi bien sous forme sèche qu'après réhydratation dans un fluide physiologique, et exempt de toxicité chimique.

Un autre objectif encore de l'invention est de fournir un patch de chirurgie viscérale réalisé à partir d'un biomatériau uniquement composé de collagène et éventuellement de gélatine, et qui soit facilement colonisable par des cellules de l'organisme.

Un autre objectif de l'invention est de fournir un patch de chirurgie viscérale réalisé à partir d'un biomatériau parfaitement toléré par le patient.

Un autre objectif encore de l'invention est de fournir un patch de chirurgie viscérale réalisé à partir d'un biomatériau à base de collagène humain que l'on puisse obtenir en quantité suffisante.

Par chirurgie viscérale, il faut comprendre notamment la chirurgie digestive, à savoir oesophagienne, gastrique, duodénale, intestinale, rectale, pancréatique et hépato-biliaire, ainsi que la chirurgie de la trachée, de l'appareil uro-génital et la neurochirurgie.

L'invention a pour objet un patch de chirurgie viscérale, caractérisé en ce qu'il est réalisé à partir d'un biomatériau formé de deux couches de collagène, non réticulé et/ou réticulé par oxydation periodique, qui sont superposées et associées intimement, à savoir une couche poreuse de collagène fibreux obtenue à partir d'une préparation de collagène que l'on lyophilise ou traite à l'acétone, pour donner une compresse que l'on tasse, et un film de collagène et/ou de gélatine.

Une efficacité particulière est obtenue si le patch, tout en conservant des qualités mécaniques suffisantes, possède une densité totale qui ne dépasse pas sensiblement 8 mg/cm², et les patchs satisfaisant à cette condition sont préférés.

En particulier, on préfère un patch dont la couche poreuse ait une densité de l'ordre de 2 mg/cm² et le film, une densité de l'ordre de 1 mg/cm².

Par aspect fibreux, il faut comprendre l'aspect macroscopique du collagène à l'état sec après lyophilisation ou traitement à l'acétone, même si, pour certains types de collagène comme le collagène de type IV, il n'y a pas de fibres microscopiques observables après solubilisation.

Dans un mode de réalisation de l'invention, le patch est obtenu par coulage d'une solution de collagène, destinée à former le film de collagène, sur une couche fibreuse dont les formes et les dimensions correspondent à celles du patch, et dans laquelle elle s'imprègne au moins partiellement.

Dans un autre mode de réalisation de l'invention, le patch est obtenu par découpage, aux dimensions et formes du patch, d'une nappe de biomatériau réalisée par coulage d'une solution de collagène, destinée à former le film de collagène, sur une couche fibreuse de collagène, dans laquelle elle s'imprègne au moins partiellement.

Le collagène utilisé dans l'invention peut être sous une forme non réticulée ou sous une forme réticulée par réticulation douce, notamment par traitement à l'acide periodique, tel que le procédé de réticulation douce par oxydation periodique décrit dans la demande de brevet français 2 601 371. Ce procédé n'induit aucun risque de toxicité.

L'invention prévoit aussi l'utilisation d'un mélange de collagène réticulé et de collagène non réticulé.

Le choix de la qualité de collagène dépendra du niveau de biodégradabilité désiré.

Le collagène utilisé est de préférence du collagène humain de type I, III, ou IV ou leur mélange (demandes de brevet français 2 597 499 et 2 586 703 au nom de l'Institut Mérieux), aussi bien pour la couche fibreuse que pour le film sus-jacent.

Ce collagène peut être ou non oxydé conformément au procédé décrit dans la demande de brevet français 2 601 371 et utilisé seul ou en mélange avec le collagène non oxydé.

La proportion de collagène oxydé par rapport au collagène non oxydé peut aller de 0 à 100 %, étant entendu qu'une plage de proportion préférée est la suivante: 20 % de collagène oxydé et 80 % de collagène natif.

Dans un autre mode de réalisation, le film est réalisé à partir de collagène soluble enrichi en type III + I.

Dans un autre mode particulier de réalisation, le collagène est remplacé par de la gélatine (substance issue du collagène) dans le film.

De préférence, le film reproduit le plus précisément possible les conditions naturelles de transmission d'humidité et autres échanges. Plus précisément, le film est perméable aux ions et aux métabolites, y compris les gaz dissous, et est imperméable aux protéines et aux bactéries.

Dans tous les cas où le biomatériau est amené à être directement en contact avec le patient, le collagène, qu'il soit destiné à la couche fibreuse ou au film, sera de préférence d'origine humaine et notamment d'origine placentaire.

L'épaisseur du film est de préférence comprise entre 5 microns et 250 microns, et notamment entre 10 et 50 microns, par exemple, de l'ordre de 20 microns, à l'état sec. A l'état humide, cette épaisseur est de préférence comprise entre 10 microns et 500 microns et notamment entre 20 et 100 microns, par exemple de l'ordre de 40 microns.

L'épaisseur de la couche fibreuse est de préférence comprise entre 100 microns et 8 mm et, notamment, de l'ordre de 1 mm à l'état sec et est sensiblement identique à l'état humide.

De préférence, le film contient à l'état humide 15 à 70 % de collagène et à l'état sec 40 à 100 % de collagène.

De préférence, la couche fibreuse contient, à l'état humide ou à l'état sec, 2 à 30 % de collagène et de préférence 5 à 20 %.

L'invention va maintenant être décrite en détail à l'aide d'exemples non limitatifs présentant divers modes de réalisation. Bien entendu, il est possible de réaliser soit une nappe de biomatériau que l'on découpera ensuite aux dimensions et formes du patch désiré, soit directement un biomatériau aux formes et dimensions du patch. Les exemples suivants concernent cette dernière possibilité, le patch désiré étant choisi rectangulaire et de dimensions égales à 5 x 7 x 0,08 cm environ.

### Préparation de la couche fibreuse.

Partant d'une solution acide de collagène de type I, III ou I + III, on réalise une précipitation à l'aide de phosphate de sodium de préférence. Le précipité est ensuite congelé et lyophilisé. On tasse la compresse obtenue afin de réaliser la couche fibreuse qui se présente alors sous forme d'un mince feuillet déshydraté de pH neutre, poreux et très dense.

En variante, partant d'une solution neutre de collagène de type IV, pouvant contenir un agent de réticulation tel que du collagène oxydé par l'acide periodique et éventuellement de glycérine, on congèle et on lyophilise, puis on tasse la compresse obtenue afin de réaliser la couche fibreuse.

### EXEMPLE 1 :

1 volume de Na₂HPO₄ (0,2 M, pH 7,5) est mélangé à 9 volumes d'une solution acide de collagène III + I (collagène III + I préparé selon le procédé décrit dans la demande de brevet français 2 597 499) à 0,125 % en eau distillée à 20°C. Il s'ensuit une précipitation. La précipitation dure de 4 à 15 heures à 20°C et le précipité est récupéré par centrifugation.

Le précipité est ensuite ajusté à une concentration de 0,5 à 3 % en collagène et de 0,5 à 3 % en glycérine avant congélation et lyophilisation.

La compresse obtenue (de dimensions 5 x 7 x 0,8 cm) est tassée pour donner un feuillet (de dimensions 5 x 7 x 0,08 cm) fibreux, peu hydrophile.

### EXEMPLE 2 :

Dans une solution acide de collagène III + I (collagène III + I préparé selon le procédé décrit dans la demande de brevet français 2 597 499) à 0,125 % en eau distillée à 20°C, il est ajouté, sous agitation, une solution d'acide periodique de façon à obtenir une concentration finale de 0,002 M.

Après 2 heures d'agitation à température ambiante, il est ajouté du Na₂HPO₄ (0,2 M, pH 7,5) dans la proportion de 9 volumes de collagène III + I pour un volume de Na₂HPO₄. Il s'ensuit une précipitation.

La précipitation dure de 4 à 15 heures et le précipité est récupéré sur toile de Nylon ou par centrifugation.

Une étape de lavage du précipité peut être ajoutée à la suite de cette étape.

On reprend alors les étapes post-précipitation de l'exemple 1.

### EXEMPLE 3 :

Il s'agit ici de collagène bovin de type I. On reprend l'exemple 1.

### EXEMPLE 4 :

Il s'agit ici de collagène bovin de type I, réticulé par oxydation periodique. On reprend l'exemple 2.

### EXEMPLE 5 :

On part d'une solution aqueuse neutre contenant 2 % de glycérine et 2 % de collagène humain de type IV non oxydé. On congèle et lyophilise, puis on tasse la compresse obtenue.

### EXEMPLE 6 :

On part d'une solution aqueuse contenant 2 % de glycérine et 2 % de collagène, à savoir 8 volumes de collagène humain de type IV non oxydé pour 2 volumes de collagène humain de type IV oxydé. On congèle et lyophilise, puis on tasse la compresse obtenue.

### EXEMPLE 7 :

On part d'une solution aqueuse de collagène obtenue à partir d'une solution de collagène humain de type IV oxydé à 1 % à laquelle on a mélangé du collagène humain de type I + III sous forme soluble, sous forme de fibrilles ou sous forme de particules, à raison d'une concentration de 10 à 30 mg/ml. On congèle et lyophilise, puis on tasse la compresse obtenue.

### Préparation du matériau.

Sur un feuillet très dense de collagène, réticulé ou non, ci-dessus décrit, il est coulé :

### EXEMPLE 8 :

2 à 10 ml d'un mélange d'une solution aqueuse de collagène IV humain à une concentration pouvant aller jusqu'à 5 % poids/volume et d'une autre solution de collagène IV humain oxydé (collagène oxydé selon le procédé décrit dans la demande de brevet français 2 601 371) dans des proportions respectives de 0 à 100 % ; après déshydradation sous une hotte à flux laminaire stérile, un biomatériau composé uniquement de collagène est obtenu ; ce biomatériau est ensuite stérilisé ; ces étapes de déshydratation et de stérilisation ne seront pas répétées dans les exemples suivants ; elles y seront sous-entendues.

Dans les exemples suivants, les concentrations les plus faibles (0,2 à 1 %) sont préférées pour des solutions froides de collagène et les concentrations les plus fortes (1 à 5 %) peuvent être utilisées pour des solutions tièdes de gélatine.

### EXEMPLE 9 :

2 à 10 ml d'une solution de collagène IV humain non oxydé à une concentration de 0,2 à 5 % ;

### EXEMPLE 10 :

2 à 10 ml d'une solution de collagène IV humain oxydé & une concentration de 0,2 à 5 % ;

### EXEMPLE 11 :

2 à 10 ml d'une solution contenant 60 à 95 % (Volume/Volume) de collagène III + I humain à une concentration de 1 % et 40 à 5 % (Volume/Volume) de collagène IV humain oxydé ;

### EXEMPLE 12 :

2 à 10 ml d'une solution de collagène III + I humain à une concentration de 0,2 à 5 % ;

### EXEMPLE 13 :

10 ml d'une solution de collagène III + I humain oxydé (collagène oxydé selon le procédé décrit dans la demande de brevet français 2 601 371) à une concentration de 0,2 à 5 % ;

### EXEMPLE 14 :

10 ml d'une solution de collagène bovin du type I mélangé ou non dans les proportions de l'exemple 8 avec du collagène IV humain oxydé ou du collagène I bovin oxydé ;

### EXEMPLE 15 :

10 ml d'une solution de collagène bovin de type I oxydé.

Cette méthode de coulage d'une solution de collagène ou de gélatine sur une structure fibreuse déshydratée permet d'obtenir une certaine imprégnation de ladite solution dans la structure fibreuse, ce qui aboutit, après déshydratation du tout, à une liaison inter-couche très résistante mécaniquement.

Le biomatériau déshydraté et stérilisé obtenu pourra, extemporanément à son utilisation, être réhydraté dans un fluide physiologique. Cette réhydratation ne lui fait pas perdre sa résistance mécanique ni sa cohésion inter-couche. Le biomatériau obtenu est très souple, s'adaptant parfaitement à n'importe quel contour.

Différentes applications du patch de collagène en chirurgie viscérale vont être maintenant décrites en détail.

### I. Application du patch de collagène dans la prévention et le traitement des fistules digestives post-opératoires :

Les désunions anastomotiques, conséquence d'une absence de cicatrisation entre deux viscères, constituent la principale source de mortalité, de morbidité et de surcoût en chirurgie digestive. C'est dire que la méthode qui permettrait de prévenir l'apparition d'une désunion anastomotique post-opératoire constituerait un progrès considérable.
a). Etude de la cicatrisation d'une perte de substance intestinale dans un contexte favorable au processus de cicatrisation :
   Après réalisation d'une perte de substance de 1 cm de diamètre à la face antérieure du caecum de 56 rats Wistar mâles, on colmate chacune des pertes de substance à l'aide d'un patch de collagène IV d'origine humaine, obtenu par découpage d'une nappe de biomatériau de façon à former des disques de 1,5 cm de diamètre, le patch de collagène étant solidarisé aux berges de la perte de substance au moyen d'une colle biologique appliquée sur la couche fibreuse.
   Les animaux ont été sacrifiés par séries de 8 aux 5ème, 10ème, 15ème, 20ème, 30ème, 40ème et 60ème jours post-opératoires et ont fait l'objet d'une étude macroscopique à la recherche d'adhérences et d'une éventuelle rétraction, d'une étude en microscopie optique et en microscopie à balayage.
   La mortalité et la morbidité imputables à la technique chirurgicale ont été nulles. Au 5ème jour post-opératoire, le patch de collagène est bien fixé sur le caecum et colmate parfaitement la brèche. Du 5ème au 20ème jours, la perte de substance intestinale cicatrise progressivement tandis que le patch est partiellement résorbé ou éliminé dans la lumière du caecum. Au 20ème jour post-opératoire, la muqueuse apparaît reconstituée. La cicatrisation musculaire commence au 30ème jour et au 40ème jour la cicatrisation est complète.
   Cette première étape expérimentale permet d'affirmer que le patch de collagène permet la cicatrisation d'une perte de substance digestive saine non suturée.
   Parce que les problèmes de cicatrisation surviennent essentiellement dans des conditions générales et locales défavorables, l'étape ultérieure a consisté à étudier les potentialités cicatricielles du patch de collagène après création d'une fistule digestive à l'origine d'une dénutrition sévère et d'une réaction inflammatoire locale peu propice à une cicatrisation per-primam.
b). Etude de la cicatrisation d'une fistule colique :
   On crée une fistule colique persistante sur 106 rats Wistar. Chaque fistule colique est fermée à la 3ème semaine à l'aide d'un patch de collagène IV d'origine humaine, ayant la forme d'un disque de 1,5 cm de diamètre obtenu comme précédemment. La colle utilisée est une colle biologique.
   On réalise des prélèvements séquentiels aux 5ème, 10ème, 15ème, 20ème, 30ème, 40ème et 60ème jours post-opératoires.
   Certains animaux sont décédés dans les 48 premières heures après la deuxième intervention, du fait de la dénutrition importante engendrée par la fistule. Aucun des décès n'a pu être attribué à une lyse du patch. La cicatrisation est identique à celle observée en cas de perte de substance saine, mais retardée. La réépithélialisation n'est totale qu'au 30ème jour, et la cicatrisation musculaire n'est obtenue qu'au 60ème jour.
c. Etude de la cicatrisation d'une anastomose intestinale protégée par un patch dans un contexte loco-régional septique :
   L'anastomose en milieu septique est considérée comme une suture à risque et de fait condamnée par la majorité des auteurs qui prônent l'absence de suture dans un premier temps.
   112 rats sont répartis en deux groupes, le groupe témoin 1 bénéficiant d'une suture simple 12 heures après création d'une péritonite, le groupe 2 bénéficiant d'une suture protégée par un patch de collagène conforme à l'invention.
   Les résultats montrent une cicatrisation digestive parfaite dans le groupe 2.

### II. Application du patch de collagène en chirurgie biliaire :

Les fuites biliaires et les sténoses sont les principales complications redoutées après ouverture de la voie biliaire principale.

Pour ne pas risquer une fuite biliaire précoce à l'origine d'une péritonite biliaire, on effectue un drainage externe après toute ouverture de la voie biliaire principale nécessitant une suture secondaire. Cette technique est astreignante, longue puisqu'elle nécessite une hospitalisation de 14 jours, le drain n'étant enlevé qu'au 12ème jour et douloureuse pour le patient.

Un patch résistant à l'agression biliaire permettrait d'éviter cette complication.
a). Etude in vitro : résistance physique du patch de collagène à l'agression biliaire.
   Les échantillons de patch de collagène sont incubés dans la bile humaine à 37°C pendant 20 jours.
   L'étude de la résistance physique est appréciée par une étude de surface en microscopie électronique à balayage, et par des tests mécaniques relatifs à la force de rupture et à la déformation de l'échantillon placé dans un extensomètre.
   L'étude de surface n'a pas montré de modification structurale aux 5ème, 7ème, et 20ème jours post-incubation.
   Les tests mécaniques n'ont pas montré d'altération après incubation dans la bile.
b). Utilisation du patch de collagène dans la cicatrisation d'une plaie du cholédoque non suturé chez le chien.
   10 chiens ont subi une incision longitudinale de 1 cm de long du cholédoque, à 2 cm du bord supérieur du premier duodénum. Cette ouverture n'a pas été suturée mais simplement colmatée par collage d'un patch de collagène réalisé directement aux formes et dimensions appropriées.
   Les chiens ont été sacrifiés au 30ème jour post-opératoire après une cholangiographie intraveineuse. La zone collée a été prélevée et chaque prélèvement a fait l'objet d'une étude macroscopique, d'une étude en microscopie optique et en microscopie électronique à balayage.
   Il a ainsi été montré que ce biomatériau permet d'assurer la bilistase, évitant toute fuite biliaire post-opératoire et aboutit à une cicatrisation cholédocienne sans ischémie pariétale.

### III. Application du patch de collagène en chirurgie hépatique :

La technique d'hépatectomie n'est pas exempte de complications telles que hémorragie, fuite biliaire et abcès sous-phrénique.

Le protocole expérimental a porté sur le rat Wistar mâle. Sur chaque rat, il a été réalisé une section hépatique sur un lobe et une ligature sélective du canal hépatique gauche.

Dans un premier groupe, le groupe témoin, la tranche de la section hépatique gauche a été protégée par de la colle biologique, sans autre geste d'hémostase et de bilistase.

Dans un deuxième groupe, la tranche de la section hépatique a été protégée par un patch de collagène, collé à l'aide de la colle biologique.

Chaque série comporte 40 animaux qui sont sacrifiés de façon séquentielle aux 5ème, 10ème, 20ème et 30ème jours post-opératoires, à raison de dix animaux par période. Chaque pièce d'hépatectomie a fait l'objet d'une étude macroscopique, d'une étude en microscopie optique et d'une étude en microscopie électronique à balayage, afin d'étudier l'intégration du patch ainsi que l'aspect de la cicatrisation. A la différence de ce qui a été observé pour le premier groupe, le deuxième groupe n'a présenté aucune fuite biliaire et sanguine. La cicatrisation des canaux biliaires sectionnés au niveau de la tranche de section a été parfaite.

Le patch de collagène a donc prévenu la fistule biliaire après l'hépatectomie.

## Revendications

1. Patch de chirurgie viscérale, caractérisé en ce qu'il est réalisé à partir d'un biomatériau formé de deux couches de collagène, non réticulé et/ou réticulé par oxydation periodique, qui sont superposées et associées intimement, à savoir une couche poreuse de collagène fibreux obtenue à partir d'une préparation de collagène que l'on lyophilise ou traite à l'acétone, pour donner une compresse que l'on tasse, et un film de collagène et/ou de gélatine.

2. Patch selon la revendication 1 caractérisé en ce qu'il possède une densité ne dépassant pas 8 mg/cm².

3. Patch selon la revendication 2, caractérisé en ce que la couche poreuse et le film présentent des densités respectives de 2 et 1 mg/cm².

4. Patch selon l'une des revendications 1 à 3, caractérisé en ce qu'il est obtenu par coulage d'une solution de collagène destinée à former le film de collagène, sur une couche poreuse de collagène fibreux dont les formes et les dimensions correspondent à celles du patch, et dans laquelle elle s'imprègne au moins partiellement.

5. Patch selon l'une des revendications 1 à 3, caractérisé en ce qu'il est obtenu par découpage, aux formes et dimensions du patch, d'une nappe de biomatériau réalisée par coulage d'une solution de collagène, destinée à former le film de collagène, sur une couche poreuse de collagène fibreux, dans laquelle elle s'imprègne au moins partiellement.

6. Patch selon l'une quelconque des revendications 1 à 5, caractérisé en ce que les proportions de collagène non réticulé et de collagène réticulé sont respectivement 80 % et 20 %.

7. Patch selon l'une quelconque des revendications 1 à 6, caractérisé en ce que la couche poreuse se compose de collagène fibreux humain de type III + I.

8. Patch selon l'une quelconque des revendications 1 à 6, caractérisé en ce que la couche poreuse se compose de collagène fibreux humain de type IV.

9. Patch selon l'une quelconque des revendications 1 à 6, caractérisé en ce que la couche poreuse se compose d'un mélange de collagène fibreux humain de type III, I et IV.

10. Patch selon l'une quelconque des revendications 1 à 6, caractérisé en ce que le film de collagène se compose de collagène humain de type IV et/ou de type III + I.

11. Patch selon l'une quelconque des revendications 1 à 6, caractérisé en ce que le film est composé de gélatine.

12. Patch selon l'une quelconque des revendications précédentes, caractérisé en ce qu'à l'état sec, l'épaisseur de la couche poreuse est comprise entre 100 microns et 8 mm.

13. Patch selon l'une quelconque des revendications précédentes, caractérisé en ce qu'à l'état sec, l'épaisseur du film est comprise entre 5 et 250 microns, notamment entre 10 et 50 microns.

## Claims

1. A visceral surgery patch, characterized in that it is made from a biomaterial made up of two superimposed and intimately associated layers of non-cured collagen and/or of collagen which has been cured by periodic oxidation, that is to say a porous layer of fibrous collagen obtained from a collagen preparation which is freeze-dried or treated with aceton for providing a compress which is packed down, and a collagen and/or gelatin film.

2. A patch according to claim 1, characterized in that it has a density of at most 8 mg/cm².

3. A patch according to claim 2, characterized in that the porous layer and the film have respective densities of 2 and 1 mg/cm².

4. A patch according to any of claims 1-3, characterized in that it is obtained by casting a collagen solution for forming the collagen film, onto a fibrous layer of collagen whose shapes and dimensions correspond to those of the patch, and into which it is at least partially impregnated.

5. A patch according to any of claims 1-3, characterized in that it is obtained by cutting out, with the shapes and dimensions of the patch, a biomaterial sheet made by casting a collagen solution, in order to form the collagen film, onto a fibrous layer of collagen, into which it is at least partially impregnated.

6. A patch according to any of claims 1-5, characterized in that the proportions of non-cured collagen to cured collagen are 80:20 % respectively.

7. Patch according to any of claims 1-6, characterized in that the fibrous layer is made up of human type III + I fibrous collagen.

8. A patch according to any of claims 1-6, characterized in that the fibrous layer is made up of human type IV fibrous collagen.

9. A patch according to any of claims 1-6, characterized in that the fibrous layer is made up of a mixture of human type III, I and IV fibrous collagen.

10. A patch according to any of claims 1-6, characterized in that the collagen film is made up of human type IV and/or type III + I collagen.

11. A patch according to any of claims 1-6, characterized in that the film is made up of gelatin.

12. A patch according to any of preceding claims, characterized in that in the dry state the thickness of the fibrous layer is between 100 microns and 8 mm.

13. A patch according to any of preceding claims, characterized in that in the dry state the film thickness is between 5-250 microns, notably between 10-50 microns.

## Patentansprüche

1. Patch für Eingeweidechirurgie, dadurch gekennzeichnet, daß es ausgehend von einem Biomaterial hergestellt wird, welches aus zwei schichten von nicht-retikuliertem und/oder durch periodische Oxidation retikuliertem Kollagen, die übereinandergelagert und innig verbunden sind, nämlich einer porösen Schicht aus faserigem Kollagen, die ausgehend von einem Kollagenpräparat, welches man lyophilisiert oder mit Aceton behandelt, um eine Kompresse bereitzustellen, die man zusammendrückt, erhalten wird, und einem Film aus Kollagen und/oder Gelatine, gebildet ist.

2. Patch nach Anspruch 1, dadurch gekennzeichnet, daß es eine Dichte, die 8 mg/cm nicht überschreitet, besitzt.

3. Patch nach Anspruch 2, dadurch gekennzeichnet, daß die poröse Schicht und der Film Dichten von 2 bzw. 1 mg/cm² aufweisen.

4. Patch nach einem der Ansprüche 1 bis 3, dadurch gekennzeichnet, daß es durch Gießen einer zur Bildung des Films aus Kollagen bestimmten Kollagenlösung auf eine poröse Schicht aus faserigem Kollagen, deren Formen und Dimensionen denen des Patches entsprechen und in die sie sich zumindest teilweise einimprägniert, erhalten wird.

5. Patch nach einem der Ansprüche 1 bis 3, dadurch gekennzeichnet, daß es durch Zerschneiden eines Vlieses aus Biomaterial, hergestellt durch Gießen einer zur Bildung des Films aus Kollagen bestimmten Kollagenlösung auf eine poröse Schicht aus faserigem Kollagen, in die sie sich zumindest teilweise einimprägniert, in die Formen und Dimensionen des Patches erhalten wird.

6. Patch nach irgendeinem der Ansprüche 1 bis 5, dadurch gekennzeichnet, daß die Anteile des nicht-retikulierten Kollagens und des retikulierten Kollagens 80% bzw. 20% sind.

7. Patch nach irgendeinem der Ansprüche 1 bis 6, dadurch gekennzeichnet, daß sich die poröse Schicht aus faserigem Human-Kollagen vom Typ III + I zusammensetzt.

8. Patch nach irgendeinem der Ansprüche 1 bis 6, dadurch gekennzeichnet, daß sich die poröse Schicht aus faserigem Human-Kollagen vom Typ IV zusammensetzt.

9. Patch nach irgendeinem der Ansprüche 1 bis 6, dadurch gekennzeichnet, daß sich die poröse Schicht aus einer Mischung aus aus faserigem Human-Kollagen vom Typ III, I und IV zusammensetzt.

10. Patch nach irgendeinem der Ansprüche 1 bis 6, dadurch gekennzeichnet, daß sich der Film aus Kollagen aus Human-Kollagen vom Typ IV und/oder vom Typ III + I zusammensetzt.

11. Patch nach irgendeinem der Ansprüche 1 bis 6, dadurch gekennzeichnet, daß der Film aus Gelatine zusammengesetzt ist.

12. Patch nach irgendeinem der vorangehenden Ansprüche, dadurch gekennzeichnet, daß die Dicke der porösen Schicht im trockenen Zustand zwischen 100 µm und 8 mm eingeschlossen liegt.

13. Patch nach irgendeinem der vorangehenden Ansprüche, dadurch gekennzeichnet, daß die Dicke des Films im trockenen Zustand zwischen 5 und 250 µm, insbesondere zwischen 10 und 50 µm, eingeschlossen liegt.
